# FASCICULE DE BREVET EUROPEEN

(11) **EP 0 402 205 B1**
(45) Date de publication et mention de la délivrance du brevet: **13.12.1995**
(21) Numéro de dépôt: 90401459.4
(22) Date de dépôt: 31.05.1990
(51) Int. Cl.: C07K 1/36, C07K 14/765, A61K 38/38

(54) **Procédé de préparation de solutions d'albumine purifiée**
Verfahren zur Herstellung von gereinigten Albuminlösungen
Process for the preparation of purified albumin solutions

(30) Priorité: 08.06.1989 FR 8907586
(43) Date de publication de la demande: 12.12.1990
(73) Titulaire: CENTRE REGIONAL DE TRANSFUSION SANGUINE DE LILLE, 59012 Lille (FR)
(72) Inventeur: Dernis, Dominique, F-59520 Marquette-Lez-Lille (FR); Burnouf, Thierry, F-59136 Wavrin (FR)
(74) Mandataire: Lepeudry-Gautherat, Thérèse

(56) Documents cités:
- EP-A- 0 050 061
- FR-A- 2 322 871
- FR-A- 2 327 256
- US-A- 4 228 154

## Description

L'invention concerne la préparation de solutions d'albumine purifiées à partir d'une solution physiologique humaine ou animale, notamment par purification par chromatographie d'échange d'ions.

On connaît depuis de nombreuses années différents types de procédés pour préparer des solutions d'albumine injectables, à partir du sérum normal. Ceux-ci comprennent la précipitation à l'éthanol, suivant les méthodes classiques de Cohn ou de Kistler et Nitschmann, et différents schémas de purification comprenant plusieurs types de colonnes de chromatographie. Le matériau de départ peut être un plasma frais ou congelé, ou un surnageant de cryoprécipité, ou une fraction déjà dépourvue de certaines protéines.

La mise au point d'un schéma de purification hautement performant a été décrite par Curling, Berglöf, Lindquist et Eriksson (Vox Sanguinis 33, 1977, 97-107 et dans le brevet US. 4 086 222). Cette technologie développée en particulier par la Société Pharmacia (Pharmacia Fine Chemicals - Uppsala - Suède) qui fournit les différents types de résines de chromatographie, est largement utilisée dans plusieurs centres de transfusion sanguine (en Europe, au Canada, en Australie ...)

Toutefois ces procédés de purification n'assurent pas l'élimination totale de certaines molécules contaminantes de nature lipoprotéique, en particulier les α-lipoprotéines, en raison de leurs analogies biochimiques avec l'albumine.

Ces molécules se révèlent instables au chauffage et semblent responsables, après dénaturation, de l'apparition soit de particules soit d'une certaine turbidité dans les solutions d'albumine au cours de la pasteurisation, ce qui les rend impropres à l'utilisation clinique. Ces contaminants sont difficiles à éliminer par des méthodes classiques basées sur des différences de charges ou de poids moléculaires.

C'est pourquoi la Demanderesse a mis au point un procédé d'extraction et d'élimination de ces contaminants lipoprotéiques, plus efficace que les étapes chromatographiques additionnelles décrites dans l'art antérieur. Ce procédé comprend une étape de délipidation de la solution d'albumine utilisant de préférence un détergent anionique non dénaturant.

Le procédé selon la présente invention comprend également préférentiellement deux étapes de séparation par chromatographie sur résine échangeuse d'ions et élution de l'albumine adsorbée, la première assurant une séparation classique de l'albumine, la seconde permettant d'éliminer le détergent et les produits résultant de la délipidation et de récupérer une solution d'albumine hautement purifiée. Celle-ci offre l'avantage d'être stable à la pasteurisation et à la conservation.

Un procédé particulier d'extraction des lipoprotéines par la silice fumée, applicable au "surnageant de Cohn", avait déjà été décrit dans le brevet US 4,228,154.

Le traitement de délipidation de la présente invention consiste en un contact prolongé de la solution d'albumine éluée de la première colonne de chromatographie avec un détergent anionique. Ce détergent peut être choisi parmi les détergents du type Tween ou Triton. En particulier, on a obtenu de très bons résultats avec le Tween 80 quand sa concentration est comprise entre 0,05 et 0,25 g par g de protéines et ajustée à 1 volume de Tween 80 pour 8 volumes de solution d'albumine.

Ce traitement de délipidation peut être mis en oeuvre par un contact assez long, plus particulièrement de plus de 5 heures, à 25°C ou par un contact plus court mais à température élevée, plus particulièrement un contact de 1 heure à 60°C. Dans ce dernier cas il est nécessaire d'ajouter un stabilisant de l'albumine comme le caprylate de sodium.

Le procédé de la présente invention peut être mis en oeuvre sur une solution physiologique quelconque, humaine ou animale, qui contient de l'albumine, comme un plasma total frais ou congelé, ou un surnageant de cryoprécipité ; on peut aussi utiliser un surnageant après précipitation du plasma à l'éthanol selon les méthodes de Cohn ou de Kistler et Nitschmann ou encore une autre fraction de plasma dont on a déjà prélevé d'autres protéines utiles. Le matériau de départ peut aussi être dérivé du placenta.

Avant d'être appliquée sur la première colonne de chromatographie, cette solution de départ contenant l'albumine est ajustée à un taux de protéines compris entre 15 et 18 g/l, à une conductivité de 1,5 à 2 mS ( milli-Siemens) par de l'acétate de sodium et à un pH compris entre 5,0 et 8,0 par l'acide acétique ou la soude.

La solution d'albumine ainsi ajustée est appliquée sur une première colonne de chromatographie sur résine échangeuse d'ions. On obtient une bonne adsorption sélective de l'albumine avec le DEAE-sépharose, par exemple sur une colonne de "DEAE-sépharose CL-6B fast flow" fourni par Pharmacia.

L'albumine adsorbée sur la colonne de chromatographie est éluée par un abaissement du pH du tampon à une valeur au moins inférieure au pHᵢ (point isoélectrique, environ égal à 4,7) et plus particulièrement comprise entre 4,0 et 4,7.

Le procédé de la présente invention comprend, après le traitement de délipidation, une séparation sur une deuxième colonne de chromatographie, de même nature et dans les mêmes conditions que la première.

Cette seconde chromatographie permet une élimination du Tween et des contaminants lipoprotéiques. Pour assurer leur élimination complète, la colonne portant l'albumine adsorbée subit plusieurs lavages successifs avec du tampon, avant l'élution de l'albumine par abaissement du pH à une valeur de 4 à 4,7, comme décrit plus haut.

La solution d'albumine éluée est ensuite ajustée à une concentration finale comprise entre 4 et 25 % dans des conditions physiologiques compatibles avec un usage thérapeutique et plus particulièrement à un pH compris entre 6,5 et 7,0 et à une osmolarité de 80 à 350 mosM.

Le procédé selon la présente invention comprend ensuite une pasteurisation de la solution d'albumine préalablement stabilisée par addition de caprylate de sodium à une concentration de 0,012 à 0,015 g/g d'albumine. Cette pasteurisation est effectuée par un chauffage à plus de 60°C pendant au moins 10 heures. Après ce traitement, les solutions restent parfaitement claires et sont stables à la conservation.

Selon un autre mode de réalisation de l'invention, la solution d'albumine ne subit pas de pasteurisation et n'est pas additionnée de caprylate de sodium ; dans ce cas, elle subit un traitement d'inactivation virale, selon des méthodes classiques, par solvant-détergent.

La présente invention concerne également les solutions d'albumine purifiée par le procédé décrit plus haut, lesdites solutions étant particulièrement adaptées à un usage thérapeutique.

Les solutions d'albumine purifiée non pasteurisées peuvent également être utilisées avantageusement pour les cultures in vitro de cellules eucaryotes.

Les exemples suivants illustrent l'invention sans toutefois en limiter la portée.

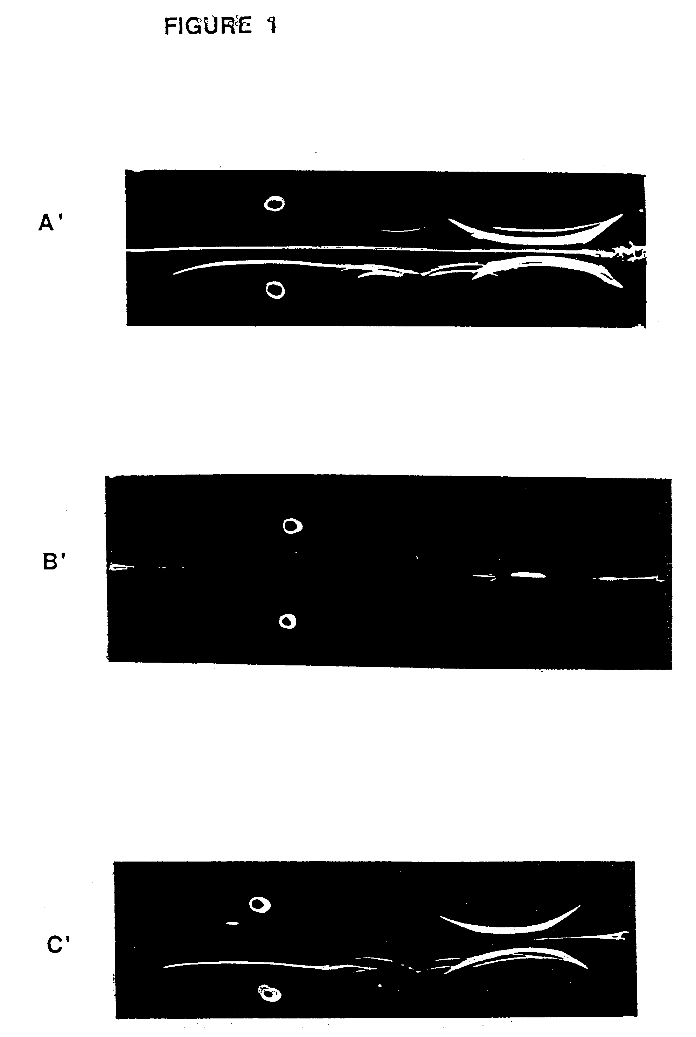

La figure 1 illustre ces exemples et représente des immunoélectrophorèses de la solution d'albumine avant et après le traitement de délipidation.

### EXEMPLE 1 :

Le matériau de départ est par exemple un surnageant I + II + III de Cohn (après précipitation du plasma à l'éthanol selon la méthode classique de Cohn et al. J. Am. Chem.Soc. 68, 1946, 459-475) ou son équivalent A + I selon la méthode classique de Kistler et Nitschmann (Vox Sanguinis 7, 1962, 414-424). Ce matériau est dialysé contre de l'eau distillée osmosée (de qualité "préparation pour injection").

La solution est ajustée à une concentration en protéines de 15 à 18 g par litre.

Le pH est ajusté à 6,0 ou à une valeur comprise entre 5,0 et 8,0 en fonction du matériau de départ, avec de l'acide acétique ou de la soude. La conductivité de la solution est ajustée à 1,8 mS ou à une valeur comprise entre 1,5 et 2,0 mS, par de l'acétate de sodium.

### a - Chromatographie

La solution est soumise à une première étape de chromatographie sur colonne de DEAE-sépharose CL6B - fast flow (Pharmacia). La colonne de chromatographie est équilibrée par un tampon d'acétate de sodium à pH 6,0. L'équilibrage du gel est atteint lorsque le tampon qui s'écoule de la colonne présente une conductivité de 1,8 ± 0,1 mS et un pH de 6,0 ± 0,1.

L'échantillon est injecté, à raison de 465 g de protéines pour 20 litres de gel. Le débit est réglé à environ 100 l/h. Après passage de l'échantillon, le retour à la ligne de base est assuré par le tampon d'équilibrage.

Ensuite, un abaissement du pH à 5,25 permet la désorption et l'élution de la transferrine.

L''albumine est ensuite éluée de la colonne par un abaissement du pH à une valeur au moins inférieure à son pHᵢ (4,7); le tampon acétate est ajusté à un pH de 4,5 et une conductivité de 1,8 mS.

La solution d'albumine éluée est concentrée à environ 100 à 150 g/l puis dialysée contre de l'eau pour éliminer l'acétate.

La solution d'albumine est ensuite ajustée à un pH de 7,0 et une osmolarité de 250 à 350 mosM ; sa concentration est ajustée soit à 4 ou 5 % soit à 20 ou 25 %.

### b. Traitement de délipidation

La solution d'albumine est additionnée de caprylate de sodium à raison de 0,015 g de caprylate par g de protéines, comme agent stabilisateur pour le chauffage et la pasteurisation ultérieurs (quand ces opérations ne doivent pas être effectuées, on n'ajoute pas de caprylate). La solution est passée sur un filtre stérilisant.

Le Tween 80 est dilué à 10 % dans de l'eau stérile chaude pour être incorporé dans la solution d'albumine à raison de 0,5 % (v/v) pour une solution d'albumine à 4 %, ou de 2,5 % (v/v) pour une solution d'albumine à 20 % (les v/v sont exprimés en v de Tween pur/vol. de solution d'albumine).

Ensuite le mélange est - soit chauffé à 60°C pendant au moins une heure - soit laissé en contact, sous légère agitation, à 25°C pendant au moins 5 heures.

### c. Chromatographie

On utilise une colonne de DEAE - sépharose CL6B dans des conditions identiques à celles utilisées pour la première étape de chromatographie

L'albumine s'adsorbe sur la colonne et le Tween 80 est éliminé dans le filtrat, en même temps que les composés de nature lipidique comme l'α-lipoprotéine.

La colonne est rincée avec 10 fois son volume de charge de tampon pour assurer l'élimination complète du Tween 80.

L'albumine est éluée dans les mêmes conditions que précédemment, par abaissement du pH du tampon à 4,5.

La solution d'albumine éluée est ensuite ajustée à des conditions physiologiques c'est-à-dire à un pH de 6,5 à 7,0 et une osmolarité de 80 à 350 mosM. La solution est filtrée, répartie en flacons et stérilisée par un chauffage à plus de 60°C pendant au moins 10 heures (selon les règles imposées par la pharmacopée).

Cette pasteurisation peut être remplacée par un traitement classique d'inactivation virale au solvent-détergent.

### EXEMPLE 2 : Mise en évidence de l'efficacité du procédé par analyse en immunoélectrophorèse

La qualité de la solution d'albumine finale est comparée à celle de la solution issue de la première colonne de chromatographie. Le résultat est présenté sur la figure 1.
- La figure 1: représente 3 immunoélectrophorèses de solutions d'albumine (dans la partie supérieure) en parallèle avec un plasma témoin (dans la partie inférieure).
1A : immunoélectrophorèse en présence d'un antisérum anti-plasma humain total, avant traitement de la solution d'albumine au Tween 80.
1B : immunoélectrophorèse en présence d'un antisérum anti-plasma humain total, après traitement de la solution d'albumine au Tween 80.
1C : même électrophorèse qu'en A, en présence d'un immunsérum spécifique anti-ApoA.
La figure 1C doit être comparée à la figure 1A et permet d'identifier l'arc d'α-lipoprotéine.
La figure 1B montre la disparition de l'arc d'α-lipoprotéine dans la solution après traitement au Tween 80.

## Revendications

1. Procédé de préparation d'albumine purifiée à partir d'une solution physiologique humaine ou animale en contenant, caractérisé en ce qu'il comprend un traitement de délipidation réalisé avec un détergent anionique non dénaturant et que ledit traitement est mis en oeuvre sur l'albumine éluée d'une première chromatographie sur résine échangeuse d'ions.

2. Procédé selon la revendication 1, caractérisé en ce qu'il comprend une deuxième étape de séparation par chromatographie sur résine échangeuse d'ions et élution de l'albumine adsorbée.

3. Procédé selon la revendication 1, caractérisé en ce que le traitement de délipidation consiste en un contact de plus de cinq heures à 25°C avec le détergent.

4. Procédé selon la revendication 1, caractérisé en ce que le traitement de délipidation consiste en un contact d'environ 1 heure à 60°C avec le détergent et en présence de caprylate de sodium comme stabilisant.

5. Procédé selon la revendication 3 ou 4, caractérisé en ce que le détergent est du Tween 80 à une concentration comprise entre 0,05 g et 0,25 g par g de protéines.

6. Procédé selon la revendication 5, caractérisé en ce que la concentration du Tween 80 est ajustée à un rapport de 1 volume de Tween 80 pour 8 volumes d'albumine.

7. Procédé selon l'une quelconque des revendications 1 à 6, caractérisé en ce que la solution physiologique de départ est un plasma total ou une fraction de plasma surnageant de cryoprécipité ou surnageant après précipitation à l'éthanol, ou toute autre fraction plasmatique contenant de l'albumine.

8. Procédé selon la revendication 7, caractérisé en ce que la solution de départ est ajustée à un taux de protéines compris entre 15 et 18 g/l.

9. Procédé selon la revendication 7 ou 8, caractérisé en ce que la solution de départ est ajustée à une conductivité comprise entre 1,5 et 2 mS par l'acétate de sodium et à un pH compris entre 5,0 et 8,0 par l'acide acétique.

10. Procédé selon l'une quelconque des revendications 1 à 9, caractérisé en ce que la solution de départ est injectée sur une première colonne de chromatographie sur résine échangeuse d'ions de type DEAE-sépharose qui adsorbe l'albumine.

11. Procédé selon l'une quelconque des revendications 1 à 10, caractérisé en ce que l'albumine adsorbée sur la chromatographie est éluée par un abaissement du pH du tampon à une valeur comprise entre 4 et 4,7.

12. Procédé selon l'une quelconque des revendications 1 à 11, caractérisé en ce que la solution d'albumine ayant subi le traitement de délipidation selon les revendications 3 à 6 est injectée sur une deuxième colonne de chromatographie sur résine échangeuse d'ions de type DEAE-sépharose qui adsorbe l'albumine et élimine le Tween et les produits résultant du traitement de délipidation.

13. Procédé selon la revendication 12, caractérisé en ce que l'élimination complète du Tween et des produits résultant de la délipidation est assurée par une série de lavages successifs de la chromatographie avec du tampon.

14. Procédé selon la revendication 12 ou 13, caractérisé en ce que l'albumine adsorbée sur la chromatographie est éluée par un abaissement du pH du tampon à une valeur comprise entre 4 et 4,7.

15. Procédé selon l'une quelconque des revendications 1 à 14 caractérisé en ce que la solution d'albumine éluée est ajustée à une concentration finale comprise entre 4 et 25 %, dans des conditions physiologiques compatibles avec un usage thérapeutique.

16. Procédé selon la revendication 15, caractérisé en ce que la solution d'albumine est ajustée à un pH compris entre 6,5 et 7,0 et à une osmolarité de 80 à 350 mosM.

17. Procédé selon la revendication 15 ou 16, caractérisé en ce que la solution d'albumine est additionnée de caprylate de sodium, à une concentration de 0,012 à 0,015 g/g d'albumine, comme stabilisant pour la pasteurisation.

18. Procédé selon l'une quelconque des revendications 1 à 17, caractérisé en ce que la solution d'albumine est soumise à une pasteurisation par un chauffage à plus de 60°C pendant au moins 10 heures.

19. Procédé selon l'une quelconque des revendications 1 à 16, caractérisé en ce que la solution d'albumine est soumise à un traitement d'inactivation virale par solvant-détergent et ne subit pas de pasteurisation.

## Claims

1. Process for the preparation of purified albumin from a human or animal physiological solution containing albumin, characterised in that it comprises a delipidation treatment effected using a non-denaturing anionic detergent and in that the said treatment is carried out on the albumin eluted from a first chromatography stage on an ion-exchange resin.

2. Process according to claim 1, characterised in that it comprises a second stage of separation by chromatography on an ion-exchange resin and elution of the adsorbed albumin.

3. Process according to claim 1, characterised in that the delipidation treatment consists of contact with the detergent for more than five hours at 25°C.

4. Process according to claim 1, characterised in that the delipidation treatment consists of contact with the detergent for approximately one hour at 60°C and in the presence of sodium caprylate as a stabiliser.

5. Process according to claim 3 or 4, characterised in that the detergent is Tween 80 at a concentration of from 0.05 g to 0.25 g per g of protein.

6. Process according to claim 5, characterised in that the concentration of Tween 80 is adjusted to a ratio of 1 volume of Tween 80 to 8 volumes of albumin.

7. Process according to any one of claims 1 to 6, characterised in that the starting physiological solution is a complete plasma or a plasma fraction obtained as cryoprecipitate supernatant or as supernatant after precipitation with ethanol, or any other plasma fraction containing albumin.

8. Process according to claim 7, characterised in that the starting solution is adjusted to a protein content of from 15 to 18 g/l.

9. Process according to claim 7 or 8, characterised in that the starting solution is adjusted to a conductivity of from 1.5 to 2 mS with sodium acetate and to a pH of from 5.0 to 8.0 with acetic acid.

10. Process according to any one of claims 1 to 9, characterised in that the starting solution is injected onto a first chromatography column that uses an ion-exchange resin of the DEAE-Sepharose type that adsorbs the albumin.

11. Process according to any one of claims 1 to 10, characterised in that the albumin adsorbed on the chromatography column is eluted by lowering the pH of the buffer to a value of from 4 to 4.7.

12. Process according to any one of claims 1 to 11, characterised in that the albumin solution that has undergone the delipidation treatment according to claims 3 to 6 is injected onto a second chromatography column that uses an ion-exchange resin of the DEAE-Sepharose type that adsorbs the albumin and removes the Tween and the products resulting from the delipidation treatment.

13. Process according to claim 12, characterised in that the complete removal of the Tween and of the products resulting from the delipidation is effected by a series of successive washings of the chromatography column using buffer solution.

14. Process according to claim 12 or 13, characterised in that the albumin adsorbed on the chromatography column is eluted by lowering the pH of the buffer to a value of from 4 to 4.7.

15. Process according to any one of claims 1 to 14, characterised in that the eluted albumin solution is adjusted to a final concentration of from 4 to 25%, under physiological conditions compatible with therapeutic use.

16. Process according to claim 15, characterised in that the albumin solution is adjusted to a pH of from 6.5 to 7.0 and to an osmolarity of from 80 to 350 mosM.

17. Process according to claim 15 or 16, characterised in that there is added to the albumin solution sodium caprylate in a concentration of from 0.012 to 0.015 g/g of albumin, as a stabiliser for pasteurisation.

18. Process according to any one of claims 1 to 17, characterised in that the albumin solution is subjected to pasteurisation by heating at more than 60°C for at least 10 hours.

19. Process according to any one of claims 1 to 16, characterised in that the albumin solution is subjected to a virus inactivation treatment using solvent-detergent and does not undergo pasteurisation.

## Patentansprüche

1. Verfahren für die Herstellung von Albumin, gereinigt aus einer dieses enthaltenden physiologischen Lösung von Mensch oder Tier, dadurch gekennzeichnet, daß es eine Lipidentfernende Behandlung umfaßt, die mit einem anionischen, nicht-denaturierenden Detergens bewerkstelligt wird, und daß die besagte Behandlung mit Albumin durchgeführt wird, das in einer ersten Chromatographie über Ionenaustauschharz eluiert wurde.

2. Verfahren nach Anspruch 1, dadurch gekennzeichnet, daß es einen zweiten Trennungsschritt durch Chromatographie über ein Ionenaustauschharz und eine Elution des adsorbierten Albumins umfaßt.

3. Verfahren nach Anspruch 1, dadurch gekennzeichnet, daß die Lipid-entfernende Behandlung aus einem mehr als 5-stündigen Kontakt mit dem Detergens bei 25°C besteht.

4. Verfahren nach Anspruch 1, dadurch gekennzeichnet, daß die Lipid-entfernende Behandlung aus einem Kontakt von ungefähr 1 Stunde mit dem Detergens bei 60°C und der Gegenwart von Natriumcaprylat als Stabilisator besteht.

5. Vefahren nach Anspruch 3 oder 4, dadurch gekennzeichnet, daß das Detergens Tween 80 in einer Konzentration zwischen 0,05 g und 0,25 g pro g Protein umfaßt.

6. Verfahren nach Anspruch 5, dadurch gekennzeichnet, daß die Konzentration von Tween 80 eingestellt wird auf ein Verhältnis von 1 Volumen Tween 80 auf 8 Volumina Albumin.

7. Verfahren nach einem der Ansprüche 1 bis 6, dadurch gekennzeichnet, daß die physiologische Ausgangslösung Gesamtplasma oder eine Plasmafraktion eines Kryopräzipitationsüberstands oder ein Überstand nach einer Ethanolpräzipitation oder jede andere Albumin-enthaltende Plasmafraktion ist.

8. Verfahren nach Anspruch 7, dadurch gekennzeichnet, daß die Ausgangslösung auf eine Proteinmenge zwischen 15 und 18 g/l eingestellt wird.

9. Verfahren nach Anspruch 7 oder 8, dadurch gekennzeichnet, daß die Ausgangslösung mit Natriumacetat auf eine Leitfähigkeit zwischen 1,5 und 2 mS und mit Essigsäure auf einen pH zwischen 5,0 und 8,0 eingestellt wird.

10. Verfahren nach einem der Ansprüche 1 bis 9, dadurch gekennzeichnet, daß die Ausgangslösung auf eine erste Chromatographiesäule mit einem Ionenaustauschharz vom Typ DEAE-Sepharose, die Albumin adsorbiert, aufgetragen wird.

11. Verfahren nach einem der Ansprüche 1 bis 10, dadurch gekennzeichnet, daß das während der Chromatographie adsorbierte Albumin durch Absenken des Puffer-pH auf einen Wert zwischen 4 und 4,7 eluiert wird.

12. Verfahren nach einem der Ansprüche 1 bis 11, dadurch gekennzeichnet, daß die Albuminlösung, die der Lipid-entfernenden Behandlung gemäß den Ansprüchen 3 bis 6 unterzogen wurde, auf eine zweite Chromatographiesäule mit Ionenaustauschharz vom Typ DEAE-Sepharose, die Albumin adsorbiert und Tween und die sich aus der Lipid-entfernenden Behandlung ergebenden Produkte entfernt, aufgetragen wird.

13. Verfahren nach Anspruch 12, dadurch gekennzeichnet, daß die vollständige Entfernung von Tween und der sich aus der Lipidentfernung ergebenden Produkte durch eine Reihe aufeinanderfolgender Chromatographie-Waschschritte mit Puffer sichergestellt wird.

14. Verfahren nach Anspruch 12 oder 13, dadurch gekennzeichnet, daß das während der Chromatographie adsorbierte Albumin durch ein Absenken des Puffer-pH auf einen Wert zwischen 4 und 4,7 eluiert wird.

15. Verfahren nach einem der Ansprüche 1 bis 14, dadurch gekennzeichnet, daß die eluierte Albuminlösung auf eine Endkonzentration zwischen 4 und 25% unter physiologischen Bedingungen, die mit einem therapeutischen Gebrauch verträglich sind, eingestellt wird.

16. Verfahren nach Anspruch 15, dadurch gekennzeichnet, daß die Albuminlösung auf einen pH zwischen 6,5 und 7,0 und eine Osmolarität von 80 bis 350 mosM eingestellt wird.

17. Verfahren nach Anspruch 15 oder 16, dadurch gekennzeichnet, daß der Albuminlösung Natriumcaprylat zu einer Konzentration von 0,012 bis 0,015 g/g Albumin als Stabilisator für die Pasteurisierung zugegeben wird.

18. Verfahren nach einem der Ansprüche 1 bis 17, dadurch gekennzeichnet, daß die Albuminlösung einer Pasteurisierung durch eine Erhitzung auf mehr als 60°C während mindestens 10 Stunden ausgesetzt wird.

19. Verfahren nach einem der Ansprüche 1 bis 16, dadurch gekennzeichnet, daß die Albuminlösung einer Behandlung zur Virusinaktivierung durch ein Lösungsmitteldetergens unterworfen und keiner Pasteurisierung unterzogen wird.
